# EUROPEAN PATENT SPECIFICATION

(11) **EP 0 398 292 B1**
(45) Date of publication and mention of the grant of the patent: **16.08.1995**
(21) Application number: 90109242.9
(22) Date of filing: 16.05.1990
(51) Int. Cl.: G01N 33/564, G01N 33/68, G01N 33/66, G01N 33/566

(54) **Diagnostic composition for rheumatoid arthritis**
Diagnostische Zusammenstellung zum Nachweis von rheumatischer Arthritis
Composition diagnostique pour l'arthrite rheumatoide

(30) Priority: 19.05.1989 JP 126177/89
(43) Date of publication of application: 22.11.1990
(73) Proprietor: Eisai Co., Ltd., Tokyo (JP)
(72) Inventor: Yamada, Yuji, Tsukuba-shi, Ibaraki (JP); Naraki, Toru, Abiko-shi, Chiba (JP); Koide, Atsushi, Tsukuba-shi, Ibaraki (JP); Mizuochi, Tsuguo, Nagoya-shi, Aichi (JP)
(74) Representative: Hansen, Bernd, Dr. Dipl.-Chem.

(56) References cited:
- EP-A- 0 058 780
- EP-A- 0 189 388
- WO-A-89/04490
- US-A- 4 617 262
- US-A- 4 863 874
- MOLECULAR IMMUNOLOGY, vol. 18, no. 12, December 1981, Oxford (GB); VAN SCHRAVENDIJK et al., pp. 1079-1085
- NATURE, vol. 316, 01 August 1985; R.B. PAREKH et al., pp. 452-457
- JOURNAL OF IMMUNOLOGY, vol. 129, no. 5, November 1982, Baltimore, MD (US); T. MIZUOCHI et al., pp. 2016-2020

## Description

The present invention relates to a diagnostic drug for rheumatoid arthritis comprising protein A or G and a labeled lectin as essential constituents which is useful in medical fields for the diagnostic of rheumatoid arthritis.

### [Prior Art]

It is known that an autoantibody called "rheumatoid factor" exists in the serum of a patient with rheumatoid arthritis (hereinafter abbreviated to "RA patient") which recognizes the antigen present in the Fc region of human immunoglobulin G (hereinafter abbreviated to "IgG") (Ref. 1). Recently, the carbohydrate chains of the Fc region of serum IgG of an RA patient have been analyzed in detail. Some of the inventors of the present invention previously found that the galactose content of the carbohydrate chains of the Fc region of serum IgG with respect to an RA patient is much lower than that with respect to a healthy man (Ref.2). That is, it has been found that the carbohydrate chains of the Fc region of serum IgG of a healthy man are constituted of various kinds of carbohydrate moieties with micro-heterogeneity without any individual difference in their proportions (Ref. 3). On the other hand, the carbohydrate chain of the Fc region of serum IgG of an RA patient has a remarkably lowered galactose content in total, though it is constituted of various kinds of carbohydrate chains with micro-heterogeneity without any individual difference in their proportions in common with healthy volunteers. More particularly, it has been found that three kinds of chains each contacting two, one or no galactose molecules exist in the Fc region of serum IgG of healthy volunteers at a ratio of 2 : 2 : 1, while the content of the carbohydrate chains containing two galactose molecules of serum IgG from patients with RA is remarkably reduced and the total content of galactose-deficient carbohydrate chains greatly increased (Ref. 2). These results suggest that the carbohydrate chains of serum IgG from patients with RA structurally cause abnormality, so that the IgG will serve as a marker for RA, if the structural abnormality can be clarified (Ref. 4).

The following References 1 to 4 disclose the above finding in detail and are consulted in the present invention.

### References:

1) Kunkel, H. G., and Tan, E. M. (1964): Autoantibodies and disease. Adv. Immunol., 4 : 351.
2) Mizuochi, T., Taniguchi, T., Matsuta, K., et al. (1985): Association of rheumatoid arthritis and primary osteoarthritis with changes in the glycosylation pattern of total serum IgG. Nature, 316,452-457.
3) Mizuochi, T., et al (1982): J. Immunol. 129, 2016-2020.
4) Mizuochi, T.(1985): Reactant to rheumatoid factor: abnormality in the sugar chains of IgG in patients with rheumatoid arthritis, Clin. Immunol. 17, 977-984.

As described above, it has been already known that the diagnosis of RA will be possible if the galactose deficiency in the carbohydrate chains of the Fc region of serum IgG can be determined. However, no practical diagnostic drug for determining the galactose deficiency has been found as yet.

PCT application WO 89/04490 discloses a method of detecting and/or measuring the terminal glycosylation of a protein by estimating the relative proportions of different terminal sugars which may be present in the protein. It is revealed that proteins bind strongly to certain solid substrates especially nitrocellulose. The bound samples are treated with labelled lectin.

European application EP-A-0189388 reveals diagnostic methods for diseases having an arthritic component. Among others, the determination of the percentage of Ig-N-linked oligosaccharide structures carrying an outer-arm galactose is disclosed. This can, for example, be done with the lectin assays for galactose.

European application EP-A-0058780 reveals a method for the determination of antigens or antibodies. This reference discloses the use of protein A for binding immunoglobulin.

Under these circumstances, the inventors of the present invention have thought that the problem to be solved by the invention resides in providing a diagnostic drug for determining the galactose deficiency in the carbohydrate chains of IgG present in human serum.

### [Summary of the invention]

The inventors of the present invention have extensively studied to solve the above problem and have found that the object is attained by adding protein A or G to serum, followed by the addition of a labeled lectin. The present invention has been accomplished on the basis of this finding. Namely, the present invention relates to a diagnostic drug for rheumatoid arthritis comprising protein A or G and a labeled lectin as essential constitutents. That is, the invention relates to a set of the two agents, protein A or G and a labeled lectin, with which galactose deficiency can be determined.

The invention provides a method for determining galactose deficiency by using the diagostic drug as defined above, which comprises the steps of reacting protein A or protein G with serum, reacting the resultant with a labeled lectin and making a color development.

The present invention will now be described in more detail.

The term "rheumatoid arthritis" used in this specification refers to autoimmune diseases and a particular sympton thereof appears as stiffness in the morning.

The term "protein A" used in this specification refers to a protein isolated from the cell membrane of Staphylococcus aureus which is constituted of a single polypeptide having a molecular weight of 42,000 and composed of some portions similar to each other in intrastructure.

Protein A has a property of binding with the Fc region of IgG, so that it is useful for the purification of IgG by virtue of this property, which itself is already known.

Like protein A, protein G is a protein isolated from Staphylococcus aureus and has a property of binding with the Fc portion of IgG so that it is useful for the purification of IgG by virtue of this property, which is also known.

The term "lectin" is a general term for carbohydrate-binding proteins. Although a lectin was available from castor seeds at first, lectins are widely distributed in animals including bacteria, so that various kinds of lectins originating from different sources are now available. Any lectin originating from any source can bind with various kinds of sugar chains different from each other in structure to recognize them. For example, many of the lectins originating from different sources can recognize a galactose-deficient mannose chain. Accordingly, the lectin to be used in the present invention is not particularly limited in origin but may be any one originating from any source. Particularly preffered examples thereof include concanavalin A and Lens culinaris agglutinin.

The labeling of the lectin as probe for detection of carbohydrate may be carried out by conventional methods, That is, it may be carried out by a process suitably selected from among those for labeling enzyme, for radio-isotope and so on. Thus, the present invention is not limited in labeling method. In the enzyme labeling, an enzyme may be bonded to a lectin either directly or through a suitable molecule such as biotin and avidin.

The determination of the galactose deficiency with the diagnostic drug of the present invention may be fundamentally carried out as follows. First, protein A is immobilized on a nitrocellulose membrane, followed by the addition of serum diluted with buffer. Then, biotin-concanavalin A solution is added to the resulting membrane to detect the carbohydrate moieties. Further, avidin-peroxidase solution is added thereto to carry out another reaction. The resulting membrane is washed and treated with a solution of a substrate against the peroxide color. Although a diagnostic drug comprising protein A and biotin-concanavalin A (labeled lectin) as essential constituents is used in the above process, the diagnostic drug according to the present invention may be suitably combined with other components to be used during the course of the operation of the determination for convenience of the user into a diagnostic drug set and examples thereof include phosphate buffer, blocking solution, nitrocellulose membrane, avidin-peroxidase, tris buffer, substrate solution and reaction stopper solution. The present invention includes such diagnostic drug sets as described above.

Although it has been already known that IgG can be purified by protein A or G and that a lectin can recognize a carbohydrate chain, combining protein A or G with a lectin into a diagnostic drug is novel, and a convenient and accurate diagnosis of RA has been realized by this composition as will be decribed in the following Experimental Examples, though it has been thought to be difficult up to this time.

### (Brief Description of the Drawings)

Fig. 1 shows the comparison of the strength of the signal generated after the staining of the serum IgG of an patient with RA with the Con A with that of a healthy volunteers. The strength of the signal was determined by the use of a Flying spotscanner CS 9000.

Fig. 2 shows the comparison of the strength of the signal generated after the staining of the serum IgG of an RA patient with LCA with that of a healthy man. The strength of the signal was determined by the use of a Flying spotscanner CS 9000.

Fig. 3 shows the amount of the serum Agalactocyl - IgG of a patient with each disease obtained in the procedure using microplate wells on the horizontal axis to develop a color, followed by the measurement of the absorbance at 405 nm and 490 nm. The names of the diseases are described in Example 2. Table 1 shows the positiveness percentage according to the results as shown in Fig. 3.

### [Example]

1. A commercially available protein A (a product of, e.g., Sigma or Pharmacia) or protein G (a product of, e.g., Sigma or Pharmacia) was combined with a labeled lectin (such as peroxidase- or biotin-labeled one) (commercially available as a product of, e.g., Sigma or Seikagaku Kogyo) to obtain a diagnostic drug according to the present invention.
2. The components described above were further combined with a solid component for immobilizing (binding) protein A (such as a nitrocellulose membrane or microplate of polystyrene), a blocking buffer (such as phosphate or Tris buffer containing BSA or gelatin), a labeled enzyme (such as streptoavidin-peroxidase), and a substrate against enzyme (such as 4-chloronaphthol) to obtain a diagnostic drug according to the present invention.
3. Protein A or G bound to or immobilized on the surface of a solid component is reacted with human plasma or serum to specifically select immunoglobulin (IgG). The carbohydrate structure present in the IgG is labeled with a labeled lectin (such as peroxidase-lectin) to detect the structural abnormality in the carbohydrate chain of the IgG by utilizing the activity of the lectin specifically recognizing the carbohydrate chain structure. The detection is carried out by determining the amount of the lectin bound to the IgG chain based on the strength of the signal such as developed color. More specifically, since the analysis of the structure of the carbohydrate chains the serum IgG of patient with RA revealed that the galactose content of the carbohydrate chains was remarkably reduced, lectins reactive with such agalactosyl IgG were screened. As the results, it was found that mannose recognizing lectins such as concanavalin A (Con A) or Lens culinaris agglutinin (LCA) are extremely reactive for agalactosyl IgG. Thus, the serum IgG of patient with RA was examined by the use of these lectins.

### Example 1

- (Sample):: Sera were prepared from patients with RA and healthy volunteers for use in the following experiments.
- (Method):: Protein A (250 »g/ml) was dissolved in a phosphate buffer (50 mM of phosphate, 0.15 M of NaCl, pH: 7.4), 10 »l of this solution was applied to a nitrocellulose membrane (dot plotting). The resulting membrane was dried and blocked with a blocking buffer (50 mM of Tris-HCl, 0.15 M of NaCl, 0.05% NP-40, 2.5% of geletin (w/v), pH: 7.4).
The resulting membrane (IgG-trapping membrane; hereinafter referred to as "IGGTM") was used in the analysis of the sugar chain of the serum IgG of patient with RA or healthy volunteers. The sera of patients with RA and healthy volunteers were diluted with a blocking buffer and added to the IGGTM to bind the IgG with the membrane (for 60 min, at room temp.) After the reaction, the IGGTM was mildly rinsed and washed for 5 minutes. Then, 500 »g/ml of biotin-Con A (20 »g/ml) was added to the resulting IGGTM to carry out a reaction (for 60 min, at room temp.). After the reaction, the resulting IGGTM was mildly rinsed and washed twice each for 5 minutes. Then, 500 »l of streptoavidin-peroxidase was added to the resulting IGGTM to carry out another reaction. After the reaction, the resulting IGGTM was mildly rinsed and washed twice each for 5 minutes. After the washing, the resulting IGGTM was mildly rinsed with TBS (20mM of Tris-HCl, 0.5 M of NaCl, pH: 7.4). A substrate solution against peroxidase (500 »g/ml) was added to the IGGTM to initiate color developement (for 5 to 10 min.). After the completion of the developement, the resulting IGGTM was sufficiently washed with distilled water and dried to measure the strength of the developed signal on the IGGTM with a Dual-wavelength Flying spotscanner CS 9000 (mfd. by Shimadzu) ( λ =540 nm).

### Result

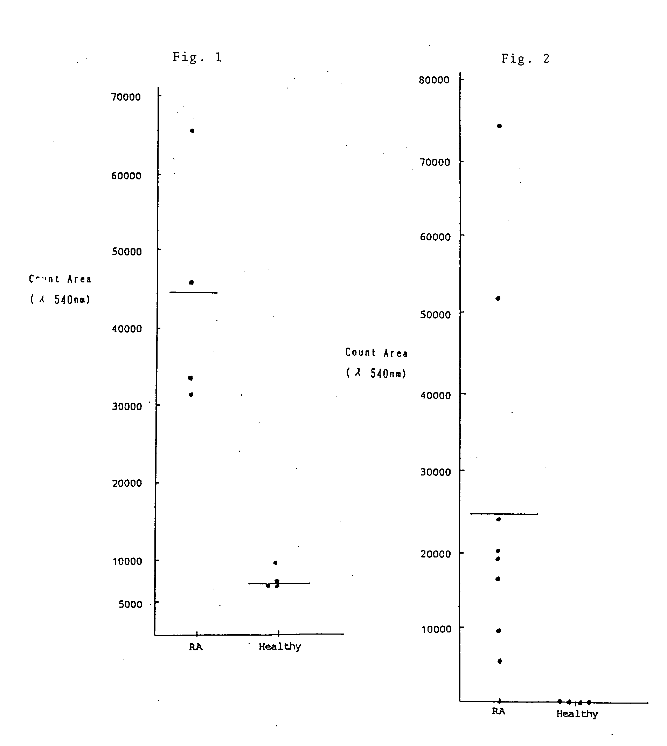

1. The serum IgG of each of four RA patients and four healthy volunteers was examined for the reactivity with Con A. The average count of the healthy group was 7603.85, while that of the RA group was 43906.22. Thus, the RA group exhibited a higher count than that of the healthy group (Fig. 1).
2. The serum IgG of each of ten patients with RA and four healthy men was examined for the reactivity with LCA. The average count of the healthy group was 0, while that of the RA group was 24501.8. Thus, the EA group exhibited a higher count than that of the healthy group (Fig.2).

### Example 2

(Sample): Sera were prepared from patients with diseases shown below and from healthy voluteers, for use in the following experiments.

| | (specimens) |
|---|---|
| Rheumatoid arthritis (RA) | 20 |
| Early phase Rheumatoid arthritis (E-RA) | 27 |
| Seronegative Rheumatoid arthritis (S-N-RA) (RA test: -, Rose, 8 or less) | 13 |
| Osteoarthritis (OA) | 18 |
| Early phase Osteoarthritis (E-OA) | 20 |
| Systemic Lupus Erthematosus (SLE) | 12 |
| Healthy volunteers | 87 |

(Method): Each microplate well was charged with 100 »l of Protein A (2.5»g / ml), and coated. It was blocked with one hundred »l of a blocking buffer (1 % BSA, 50 mM phosphate buffer, 0.15 M NaCl, pH: 7.4). One hundred »l of gelatin buffer (50m M Tris - HCl, 0.15 M NaCl, 0.05% NP-40, 2.5%(w/v) gelatin, pH: 7.4) was added to each well, followed by adding thereto 1 »l of sample. Then, to each well was added further one hundred »l of the gelatin buffer. All wells were incubated at 25°C for 60 min. After the reaction, each well was washed with washing buffer (50m M Tris - HCl, 0.15 M NaCl. 0.05% NP-40, pH: 7.4). Then, one hundred »l of biotin-Con A solution (0.0625 »g / ml) was added thereto and the reaction as carried out at 25°C for 60 min.
The reaction product was washed with the washing buffer. Then 100 »l of streptoavidin-peroxidase solution was added. A reaction was conducted at 25°C for 60 min. The reaction product was washed with the washing buffer, followed by adding 100 »l of substrate solution (3% H₂O₂, 10 »l + ABTS 6 mg / 4ml, a citric acid buffer, pH: 4.2) to carry out a reaction at 37 °C for 60 minutes.
To each well was added 100 »l of sodium azide solution to terminate the reaction and the absorbance at 405 nm and at 490 nm of the resulting specimen was measured by the use of a dual-wavelength spectrophotometer.

### Result

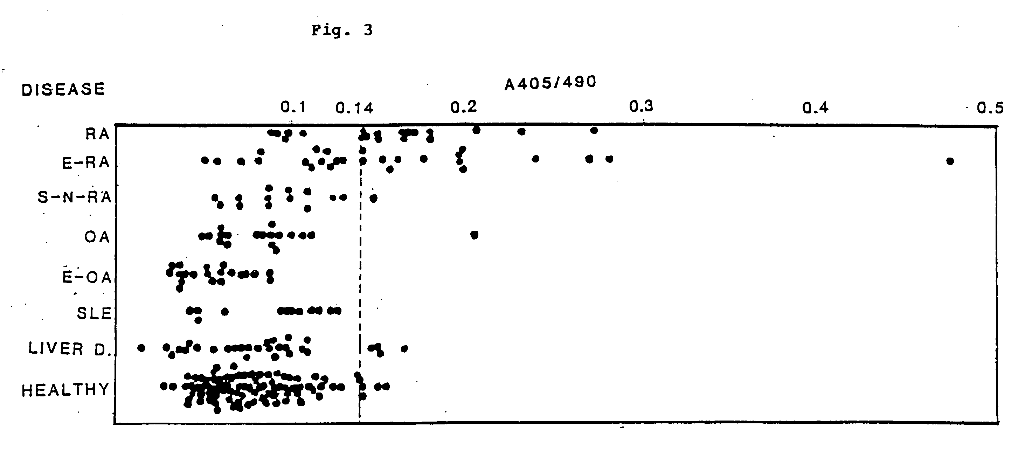

Fig. 3 shows the following.
(1) The absorbance 0.14 is defined as the cut-off value; Rheumatoid Arthritis is recognized as RA when the measured absorbance of a specimen is larger than the cut-off value. The positiveness ratio, each disease is shown in Table 1.
(2) A high positiveness ratio is recognized with respect to those diseases other than RA, i.e., SLE, OA, and LD, through a conventional RA test, such as the hemagglutination method and the latex coaggulation method, according to Tatsu ABE, General Clinic (Sogo Rinsho) 34, 1915 (1985). The assay method of the present invention provides a very low positive ratio with respect to those diseases, but a very high selectivity on the RA disease.

**Table 1**

| DISEASE | CUP (A405/490) | RATIO (%) |
|---|---|---|
| RA | 0.155±0.048 | 75(15/20) |
| E-RA | 0.158±0.048 | 53.8(14/27) |
| S-N-RA | 0.092±0.027 | 7.7(1/13) |
| OA | 0.086±0.035 | 5.6(1/18) |
| E-OA | 0.057±0.018 | 0(0/20) |
| SLE | 0.09 ±0.032 | 0(0/12) |
| LIVER D. | 0.087±0.046 | 17.2(5/29) |
| CONTROL | 0.081±0.03 | 4.6(4/87) |

## Claims (Claims for the following Contracting State(s): AT, BE, CH, LI, DE, DK, FR, GB, IT, LU, NL, SE)

1. A diagnostic composition for rheumatoid arthritis comprising protein A or G and concanavalin A or lens culinaris agglutinin as a labelled lectin as essential constituents, wherein said lectin is present for recognizing galactose-deficient sugar chains.

2. A method for determining galactose deficiency by using the diagnostic composition as defined in claim 1 which comprises the steps of reacting protein A or protein G with serum, reacting the resultant with concanavalin A or lens culinaris agglutinin as a labelled lectin and making a colour development.

3. Kit of parts for a diagnostic composition for rheumatoid arthritis, essentially consisting of
(a) a protein A or G in a first part, and
(b) concanavalin A or lens culinaris agglutinin as a labelled lectin in a second part for recognizing galactose-deficient sugar chains.

4. A kit of parts for a diagnostic composition according to claim 3, wherein said composition additionally comprises a phosphate buffer, a blocking solution, a nitrocellulose membrane, an avadin-peroxidase, a tris-buffer, a substrate solution and/or a reaction stopper solution.

## Claims (Claims for the following Contracting State(s): ES, GR)

1. A method for determining galactose deficiency by using a diagnostic composition comprising protein A or G and concanavalin A or lens culinaris agglutinin as a labelled lectin as essential constituents wherein said lectin is present for recognizing galactose-deficient sugar chains, which comprises the steps of reacting protein A or protein G with serum, reacting the resultant with concanavalin A or lens culinaris agglutinin as a labelled lectin and making a colour development.

## Patentansprüche (Patentansprüche für folgende(n) Vertragsstaat(en): AT, BE, CH, LI, DE, DK, FR, GB, IT, LU, NL, SE)

1. Diagnostische Zusammensetzung für die rheumatoide Arthritis, umfassend Protein A oder G und Concanavalin A oder Culinaris-Linsen-Agglutinin als markiertes Lectin als wesentliche Bestandteile, wobei dieses Lectin zum Erkennen von Galactose-defizienten Zuckerketten vorliegt.

2. Verfahren zur Bestimmung von Galactosemangel unter Verwendung der diagnostischen Zusammensetzung gemäss Anspruch 1, umfassend die Schritte der Umsetzung von Protein A oder Protein G mit Serum, Umsetzen des Resultierenden mit Concanavalin A oder Culinaris-Linsen-Agglutinin als markiertes Lectin, und Ausführung einer Farbentwicklung.

3. Reagenziensatz für eine diagnostische Zusammensetzung für die rheumatoide Arthritis, bestehend im wesentlichen aus
(a) Protein A oder G in einem ersten Teil und
(b) Concanavalin A oder Culinaris-Linsen-Agglutinin als markiertes Lectin in einem zweiten Teil zur Erkennung von Galactose-defizienten Zuckerketten.

4. Reagenziensatz für eine diagnostische Zusammensetzung gemäss Anspruch 3, wobei diese Zusammensetzung ausserdem einen Phosphatpuffer, eine Blockierlösung, eine Nitrocellulosemembran, eine Avidin-Peroxidase, einen Tris-Puffer, eine Substratlösung und/oder eine Reaktionsabbruchlösung umfasst.

## Patentansprüche (Patentansprüche für folgende(n) Vertragsstaat(en): ES, GR)

1. Verfahren zur Bestimmung des Galactosemangels unter Verwendung einer diagnostischen Zusammensetzung, welche Protein A oder G und Concanavalin A oder Culinaris-Linsen-Agglutinin als markiertes Lectin als wesentliche Bestandteile umfasst, wobei das Lectin vorliegt, um Galactose-defiziente Zuckerketten zu erkennen, das die Schritte der Umsetzung von Protein A oder Protein G mit Serum, Umsetzung des Resultierenden mit Concanavalin A oder Culinaris-Linsen-Agglutinin als markiertes Lectin und die Ausführung einer Farbentwicklung umfasst.

## Revendications (Revendications pour l'(les) Etat(s) contractant(s) suivant(s): AT, BE, CH, LI, DE, DK, FR, GB, IT, LU, NL, SE)

1. Composition diagnostic de l'arthrite rhumatoïde, comprenant une protéine A ou G et de la concanavaline A ou de l'agglutinine de Lens culinaris en tant que lectine marquée, comme constituants essentiels, cette lectine étant présente pour reconnaître des chaînes glucidiques déficientes en galactose.

2. Procédé de détermination d'une déficience en galactose, utilisant la composition de diagnostic selon la revendication 1, comprenant les étapes consistant à faire réagir une protéine A ou G avec un sérum, à faire réagir le produit résultant avec de la concanavaline A ou de l'agglutinine de Lens culinaris en tant que lectine marquée, et à provoquer une coloration.

3. Nécessaire pour composition diagnostique de l'arthrite rhumatoïde, consistant essentiellement :
(a) en premier lieu, en une protéine A ou G, et
(b) en deuxième lieu, en concanavaline A ou en agglutine de Lens culinaris en tant que lectine marquée, permettant de reconnaître des chaînes glucidiques déficientes en galactose.

4. Nécessaire pour composition diagnostique selon la revendication 3, cette composition comprenant en outre un tampon phosphate, une solution de blocage, une membrane de nitrocellulose, une avidine-peroxydase, un tampon tris, une solution de substrat et/ou une solution d'arrêt de réaction.

## Revendications (Revendications pour l'(les) Etat(s) contractant(s) suivant(s): ES, GR)

1. Procédé de détermination d'une déficience en galactose utilisant une composition diagnostic, comprenant une protéine A ou G et de la concanavaline A ou de l'agglutinine de Lens culinaris en tant que lectine marquée, comme constituants essentiels, cette lectine étant présente pour reconnaître des chaînes glucidiques déficientes en galactose, comprenant les étapes consistant à faire réagir une protéine A ou G avec un sérum, à faire réagir le produit résultant avec de la concanavaline A ou de l'agglutinine de Lens culinaris en tant que lectine marquée, et à provoquer une coloration.
